# EUROPEAN PATENT APPLICATION

(11) **EP 3 733 047 A1**
(43) Date of publication of application: **04.11.2020**
(21) Application number: 19750752.8
(22) Date of filing: 29.01.2019
(51) Int. Cl.: A61B 1/045, A61B 90/20

(54) **SURGICAL SYSTEM, IMAGE PROCESSING DEVICE, AND IMAGE PROCESSING METHOD**

(30) Priority: 09.02.2018 JP 2018021695
(71) Applicant: Sony Corporation, 108-0075 Tokyo (JP)
(72) Inventor: NEISHI, Hideo, Tokyo 108-0075 (JP); TSUBOI, Toshimitsu, Tokyo 108-0075 (JP); OKI, Tomoyuki, Tokyo 108-0075 (JP); SHIRAKI, Hisakazu, Tokyo 108-0075 (JP)
(74) Representative: D Young & Co LLP
(86) International application number: PCT/JP2019/002839
(87) International publication number: WO 2019/155931

(57) **Abstract**

[Problem to be Solved] Proposed is a surgery system, an image processor, and an image processing method that make it possible to know distribution of tissue inside a biological organ more exactly.

[Solution] A surgery system including a surgical camera and an image processor. The surgical camera performs imaging of a biological organ to acquire an image of the biological organ. The image processor specifies element tissue included in a three-dimensional region being a portion of the biological organ during surgery on the basis of three-dimensional distribution information of element tissue included in the biological organ and superimposes a representation of the specified element tissue on the image of the biological organ.

## Description

### Technical Field

The present disclosure relates to a surgery system, an image processor, and an image processing method.

### Background Art

In medical practice, the widespread use of surgical cameras such as endoscopes as devices for observing the interior of biological bodies has brought about more situations where a medical doctor checks an affected area inside a biological body on an image acquired using a surgical camera and performs treatment rather than directly checking the affected area with unaided eyes. In recent years, a number of technologies have been proposed to assist in surgery through image processing performed on an image acquired using a surgical camera in accordance with intended use.

As one of such surgery assisting technologies, for example, PTL 1 listed below discloses a technology to observe a vascular structure or the like that runs in a depth direction around a surface of a biological organ or the like being a subject.

### Citation List

### Patent Literature

PTL 1: Japanese Unexamined Patent Application Publication No. 2001-170009

### Summary of the Invention

### Problems to be Solved by the Invention

However, according to the technology disclosed in PTL 1, it is possible to observe tissue inside the biological organ, but it is difficult to know the exact distance thereof from the surface of the biological organ.

In view of the above-described circumstances, therefore, the present disclosure proposes a surgery system, an image processor, and an image processing method that make it possible to know distribution of tissue inside a biological organ more exactly.

### Means for Solving the Problems

The present disclosure provides a surgery system including a surgical camera and an image processor. The surgical camera performs imaging of a biological organ to acquire an image of the biological organ. The image processor specifies element tissue included in a three-dimensional region being a portion of the biological organ during surgery on the basis of three-dimensional distribution information of element tissue included in the biological organ and superimposes a representation of the specified element tissue on the image of the biological organ.

The present disclosure also provides an image processor including an image processing unit that specifies element tissue included in a three-dimensional region being a portion of a biological organ during surgery on the basis of three-dimensional distribution information of element tissue included in the biological organ and superimposes a representation of the specified element tissue on an image of the biological organ acquired by a surgical camera.

The present disclosure also provides an image processing method including implementation by a processor of specifying, element tissue included in a three-dimensional region being a portion of a biological organ during surgery on the basis of three-dimensional distribution information of element tissue included in the biological organ, and superimposing a representation of the specified element tissue on an image of the biological organ acquired by a surgical camera.

According to the present disclosure, the element tissue included in the three-dimensional region being the portion of the biological organ is specified during surgery on the basis of three-dimensional distribution information of element tissue included in the biological organ, and the representation of the specified element tissue is superimposed on the image of the biological organ.

### Effects of the Invention

According to the present disclosure, as described above, it is possible to know distribution of tissue in a biological organ more exactly.

It is to be noted that the effects described above are not necessarily limitative. With or in the place of the effects described above, there may be achieved any one of the effects described in this specification or other effects that may be grasped from this specification.

### Brief Description of Drawing

[FIG. 1] FIG. 1 is a diagram illustrating an example of a situation of surgery adopting an endoscopic surgery room system employing a technical idea according to the present disclosure.
[FIG. 2] FIG. 2 is a block diagram illustrating a configuration example of a surgery system according to an embodiment of the present disclosure.
[FIG. 3] FIG. 3 is an explanatory diagram for describing an example of a method of measuring positions in a patient according to the same embodiment.
[FIG. 4] FIG. 4 is an explanatory diagram for describing an example of a method of displaying a superimposition image according to the same embodiment.
[FIG. 5A] FIG. 5A is an explanatory diagram for describing an example of the method of displaying a superimposition image according to the same embodiment.
[FIG. 5B] FIG. 5B is an explanatory diagram for describing an example of the method of displaying a superimposition image according to the same embodiment.
[FIG. 5C] FIG. 5C is an explanatory diagram for describing an example of the method of displaying a superimposition image according to the same embodiment.
[FIG. 6] FIG. 6 is an explanatory diagram for describing an example of the method of displaying a superimposition image according to the same embodiment.
[FIG. 7] FIG. 7 is a flowchart illustrating a flow of an operation of the surgery system according to the same embodiment.
[FIG. 8] FIG. 8 is a flowchart illustrating a flow of an operation of the surgery system according to the same embodiment.
[FIG. 9] FIG. 9 is a flowchart illustrating a flow of an operation of the surgery system according to the same embodiment.
[FIG. 10] FIG. 10 is a diagram illustrating an example of a hardware configuration of a control system according to an embodiment of the present disclosure.

### Modes for Carrying Out the Invention

The following describes a preferred embodiment of the present disclosure in detail with reference to the accompanying drawings. It is to be noted that in this description and the accompanying drawings, constituent elements that have substantially the same functional configuration are indicated by the same reference signs, and thus redundant description thereof is omitted.

Furthermore, in this description and the accompanying drawings, a plurality of constituent elements that has substantially the same functional configuration may be indicated by the same reference signs followed by different alphabets to distinguish such constituent elements from one another. However, in a case where it is not necessary to particularly distinguish a plurality of constituent elements that has substantially the same functional configuration from one another, such constituent elements are only indicated by the same reference signs.

It is to be noted that the description is given in the following order.
< 1. Background>
<2. Configuration>
   <2-1. Overall Configuration>
   <2-2. Configuration of Surgery System 1>
<3. Operation>
<4. Hardware Configuration>
<5. Conclusion>

### <1. Background>

Among surgical operations, liver surgery has been considered difficult. One of reasons thereof is that liver surgery is accompanied by significant bleeding. A liver is a solid organ and has more blood vessels than a hollow organ. It is therefore likely that significant bleeding occurs and the risk of complications associated with the bleeding is high in liver excision, as compared with surgery on a hollow organ such as the digestive tract, for example. It is therefore important to keep the amount of bleeding to a minimum in liver surgery.

Meanwhile, in many cases of hepatocellular carcinoma, for example, a tumor located near a portal vein, which is a thick blood vessel through which blood flows from the digestive tract into the liver, expands due to the blood flowing through the portal vein. However, it is difficult for a surgeon to exactly know the position of the tumor and the positions of blood vessels including the portal vein during surgery. Furthermore, the surgeon performs the surgery while tensioning the liver during the surgery, which causes a change in the shape of the liver. This change in the shape makes it more difficult for the surgeon to know the position of an important blood vessel. As a result, there is a possibility that the surgeon damages a main blood vessel such as the portal vein by mistake to cause significant bleeding. One method of reducing the amount of bleeding is performed by ligating the portal vein and removing an affected area while the blood flow to the liver is temporarily decreased. However, this method imposes an enormous burden on the body of a patient and causes the liver to become necrotic in some situations.

Furthermore, open abdominal surgery used to be mainstream, but the use of laparoscopic surgery, which involves making some small incisions in an abdomen and inserting an endoscope or a surgical tool through any of the incisions, has been increasing for the purpose of reducing the burden on the body of the patient. However, the laparoscopic surgery does not allow the surgeon to directly hold the liver with a hand, and is therefore a more difficult surgical procedure than the open abdominal surgery.

In order to solve the above-described issues, a technology to know a position of an important blood vessel during surgery has been developed. For example, as described above, a technology to observe a vascular structure that runs in a depth direction around a surface of a biological organ is disclosed. However, such a technology may disadvantageously pose an issue. For example, an endoscopic image of a biological organ having a number of blood vessels, such as a liver, exhibits a number of blood vessel images thereon at once, preventing the surgeon from distinguishing an important blood vessel from less important blood vessels, and thus interfering with the surgeon's concentration. Furthermore, the above-described technology entails observation mode switching between normal observation in which a surface of the biological organ is observed and a special observation in which a vascular structure or the like that runs inside the biological organ is observed, which may be complicated work during surgery.

The present inventors have therefore conceived an idea of selectively superimposing and displaying a blood vessel located in a predetermined region of a biological organ on an endoscopic image acquired during surgery, and consequently have invented the present technology.

### <2. Configuration>

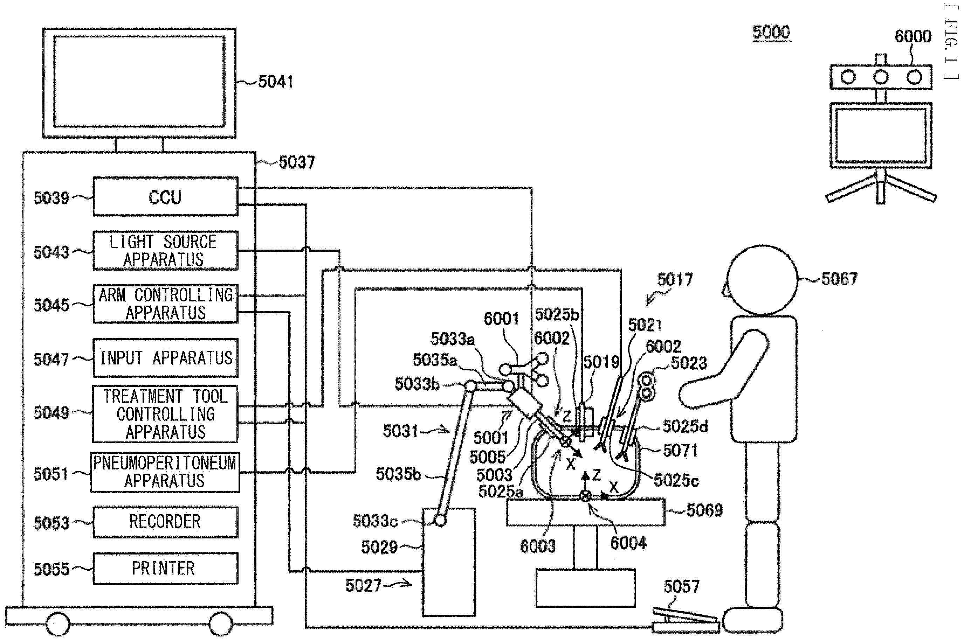

Through the above, the background for inventing the present technology has been described. The following describes a configuration of a surgery system according to the present disclosure with reference to FIGs. 1 to 6.

### <2-1. Overall Configuration>

First, a schematic configuration of an endoscopic surgery system 5000 to which the technology according to the present disclosure is applicable is described. FIG. 1 is a diagram illustrating an example of a schematic configuration of the endoscopic surgery system 5000 to which the technology according to the present disclosure is applicable. FIG. 1 illustrates a situation in which a surgeon (a medical doctor) 5067 is performing surgery on a patient 5071 on a patient bed 5069 using the endoscopic surgery system 5000. As illustrated in FIG. 1, the endoscopic surgery system 5000 includes an endoscope 5001, other surgical tools 5017, a supporting arm apparatus 5027 that supports the endoscope 5001, a cart 5037 in which various devices for endoscopic surgery are mounted, and a position measurement apparatus 6000.

In endoscopic surgery, the abdominal wall is punctured with a plurality of tubular piercing devices referred to as trocars 5025a to 5025d in place of incision of the abdominal wall to perform laparotomy. Then, a lens barrel 5003 of the endoscope 5001 and the other surgical tools 5017 are inserted into body cavity of the patient 5071 through the trocars 5025a to 5025d. In the illustrated example, as the other surgical tools 5017, a pneumoperitoneum tube 5019, an energy device 5021, and forceps 5023 are inserted into the body cavity of the patient 5071. Further, the energy device 5021 is a treatment tool that performs incision and peeling of tissue, sealing of a blood vessel or the like by high frequency current or ultrasonic vibration. However, the illustrated surgical tools 5017 are merely examples, and various surgical tools that are generally used in endoscopic surgery, such as tweezers or a retractor, may be used as the surgical tools 5017, for example.

An image of a surgical region in a body cavity of the patient 5071 captured by the endoscope 5001 is displayed on a display apparatus 5041. A surgeon 5067 performs, for example, treatment such as removal of an affected area using the energy device 5021 and the forceps 5023 while watching the image of the surgical region displayed on the display apparatus 5041 in real time. It is to be noted that during the surgery, the pneumoperitoneum tube 5019, the energy device 5021, and the forceps 5023 are held by the surgeon 5067 or another person such as an assistant, which is not illustrated.

### (Supporting Arm Apparatus 5027)

The supporting arm apparatus 5027 includes an arm unit 5031 extending from a base unit 5029. In the illustrated example, the arm unit 5031 includes joint portions 5033a, 5033b, and 5033c, and links 5035a and 5035b, is driven under the control of an arm controlling apparatus 5045. The arm unit 5031 supports the endoscope 5001 and controls the position and the posture of the endoscope 5001. This allows the endoscope 5001 to be fixed in a stable position.

### (Endoscope 5001)

The endoscope 5001 includes the lens barrel 5003 and a camera head 5005 coupled to a proximal end of the lens barrel 5003. Of the lens barrel 5003, a region having a predetermined length from a distal end of the lens barrel 5003 is inserted into a body cavity of the patient 5071. In the illustrated example, the endoscope 5001 is configured as so-called rigid scope having the rigid lens barrel 5003. However, the endoscope 5001 may be configured as a so-called flexible scope having a flexible lens barrel 5003.

The distal end of the lens barrel 5003 has an opening with an objective lens fitted therein. A light source apparatus 5043 is coupled to the endoscope 5001. Light generated by the light source apparatus 5043 is guided to the distal end of the lens barrel by a light guide extending inside the lens barrel 5003, and applied onto an observation target in the body cavity of the patient 5071 through the objective lens. It is to be noted that the endoscope 5001 may be a forward-viewing endoscope, or may be an oblique-viewing endoscope or a side-viewing endoscope.

An optical system and an imaging element are provided inside the camera head 5005. The optical system focuses reflected light (observation light) from an observation target on the imaging element. The imaging element performs photoelectric conversion on the observation light to generate an electrical signal corresponding to the observation light, which in other words is an image signal corresponding to an observation image. The image signal is transmitted to a camera control unit (CCU) 5039 as RAW data. It is to be noted that the camera head 5005 has a function of adjusting magnification and focal length through the optical system being driven as appropriate.

It is to be noted that a plurality of imaging elements may be provided in the camera head 5005 for stereoscopic vision (3D display), for example. In this case, a plurality of relay optical systems is provided inside the lens barrel 5003 to guide the observation light to each of the plurality of imaging elements.

### (Various Apparatus Mounted in Cart)

The CCU 5039 includes a CPU (Central Processing Unit), a GPU (Graphics Processing Unit), and the like, and provides overall control of operations of the endoscope 5001 and the display apparatus 5041. Specifically, the CCU 5039 performs, on the image signal received from the camera head 5005, for example, various types of image processing such as development (demosaicing) for displaying an image on the basis of the image signal. The CCU 5039 supplies the image signal that has gone through the image processing to the display apparatus 5041. The CCU 5039 also transmits a control signal to the camera head 5005 to control driving of the camera head 5005. The control signal may include information related to imaging conditions such as magnification and focal length.

The display apparatus 5041 is controlled by the CCU 5039 to display an image based on the image signal that has gone through the image processing by the CCU 5039. In a case where the endoscope 5001 is ready for imaging of high resolution such as 4K (horizontal pixel number 3840 × vertical pixel number 2160) or 8K (horizontal pixel number 7680 × vertical pixel number 4320) and/or the endoscope 5001 is ready for 3D display, for example, a display apparatus enabling high resolution display and/or 3D display may be accordingly used as the display apparatus 5041. In a case where the endoscope 5001 is ready for imaging of high resolution such as 4K or 8K, it is possible to obtain an enhanced sense of immersion by employing a 55-inch or larger display apparatus as the display apparatus 5041. Furthermore, a plurality of display apparatuses 5041 that are different in resolution and size may be provided in accordance with intended use.

The light source apparatus 5043 includes, for example, a light source such as LED (Light emitting diode) and supplies irradiation light for imaging a surgical region to the endoscope 5001.

The arm controlling apparatus 5045 includes, for example, a processor such as CPU, and operates in accordance with a predetermined program to control driving of the arm unit 5031 of the supporting arm apparatus 5027 in accordance with a predetermined control scheme.

An input apparatus 5047 is an input interface for the endoscopic surgery system 5000. Users including the surgeon 5067 are allowed to input various types of information and instructions to the endoscopic surgery system 5000 using the input apparatus 5047. For example, through the input apparatus 5047, a user inputs various types of information related to surgery such as physical information of a patient or information regarding a surgical procedure of the surgery. In addition, for example, through the input apparatus 5047, the user inputs instructions such as an instruction to drive the arm unit 5031, an instruction to change conditions for imaging by the endoscope 5001 (type of irradiation light, magnification, focal length, or the like), and an instruction to drive the energy device 5021.

The type of the input apparatus 5047 is not limited, and the input apparatus 5047 may be any of various known input apparatuses. As the input apparatus 5047, for example, a mouse, a keyboard, a touch panel, a switch, a foot switch 5057 and/or a lever, or the like may be applied. In a case where a touch panel is used as the input apparatus 5047, the touch panel may be disposed over a display surface of the display apparatus 5041.

Alternatively, the input apparatus 5047 is, for example, a device to be mounted on the user such as a glasses type wearable device or a HMD (Head Mounted Display), and various kinds of inputting are performed in response to a gesture or a line of sight of the user detected by any of the devices. Further, the input apparatus 5047 includes a camera that is able to detect the user's movement, and various types of inputting are performed in response to a gesture or a line of sight of a user detected from from an image captured by the camera. Furthermore, the input apparatus 5047 includes a microphone that is able to pick up the user's voice, and various types of inputting are performed by voice through the microphone. As described above, the input apparatus 5047 is configured to input various types of information in a contactless manner. This allows the user who belongs to a clean area (for example, the surgeon 5067), in particular, to operate a device that belongs to an unclean area in a contactless manner. In addition, this allows the user to operate the device without releasing a possessed surgical tool from his hand, thereby offering improved convenience for the user.

A treatment tool controlling apparatus 5049 controls driving of the energy device 5021 for cautery or incision of tissue, sealing of a blood vessel, or the like. A pneumoperitoneum apparatus 5051 feeds gas into a body cavity of the patient 5071 through the pneumoperitoneum tube 5019 to inflate the body cavity for the purpose of securing a field of view through the endoscope 5001 and securing work space for the surgeon 5067. A recorder 5053 is an apparatus that is able to record various types of information related to the surgery. A printer 5055 is an apparatus that is able to print various types of information related to the surgery in various formats such as a text format, an image format, or a graph format.

The position measurement apparatus 6000 measures positions and/or postures of a tool, which is to be used in the endoscopic surgery such as the endoscope 5001 or the surgical tools 5017, and the patient. The positions and/or the postures of the tool and the patient 5071 acquired by the position measurement apparatus 6000 allow for registration of three-dimensional information of element tissue acquired by an apparatus that performs imaging of the interior of a biological body onto an image acquired by the endoscope 5001 being used during the surgery. The three-dimensional information of element tissue includes a CT (Computed Tomography) image, an MRI (Magnetic Resonance Imaging) image, or an ultrasonograph image of element tissue such as a blood vessel, a nervous plexus, a lymphatic vessel, or a tumor of the patient 5071. The element tissue here refers to tissue forming a healthy biological organ, abnormal tissue formed in a biological organ, and the like. Examples thereof include a blood vessel, a nervous plexus, a lymphatic vessel, and a tumor as described above.

The following particularly describes characteristic configurations of the endoscopic surgery system 5000 in more detail.

### (Supporting arm apparatus 5027)

In endoscopic surgery, in general, a medical doctor called a scopist holds the endoscope 5001, but the position of the endoscope 5001 may be fixed using the supporting arm apparatus 5027. The supporting arm apparatus 5027 includes the base unit 5029 serving as a base and the arm unit 5031 extending from the base unit 5029. In the illustrated example, the arm unit 5031 includes the plurality of joint portions 5033a, 5033b, and 5033c, and the plurality of links 5035a and 5035b coupled to each other by the joint portion 5033b. For simplification, however, a configuration of the arm unit 5031 is illustrated in FIG. 1 in a simplified manner. Actually, the shapes, the number, and arrangement of the joint portions 5033a to 5033c and the links 5035a and 5035b, and the directions and the like of rotational axes of the joint portions 5033a to 5033c are settable as appropriate in order for the arm unit 5031 to have a desired degree of freedom. For example, the arm unit 5031 may preferably be configured to have six degrees or more of freedom. This makes it possible to move the endoscope 5001 freely within a movable range of the arm unit 5031, allowing the lens barrel 5003 of the endoscope 5001 to be inserted into the body cavity of the patient 5071 from a desired direction.

The joint portions 5033a to 5033c are provided with actuators. The joint portions 5033a to 5033c are driven by the corresponding actuators to be rotatable about predetermined rotational axes. The arm controlling apparatus 5045 controls driving of the actuators to control rotation angles of the respective joint portions 5033a to 5033c, thereby controlling the driving of the arm unit 5031. This enables control of the position and the posture of the endoscope 5001. Thereupon, the arm controlling apparatus 5045 is able to control the driving of the arm unit 5031 by any of various known control methods such as force control or position control.

For example, the surgeon 5067 may perform operation inputting through the input apparatus 5047 (including the foot switch 5057) as appropriate to cause the arm controlling apparatus 5045 to control the driving of the arm unit 5031 as appropriate in response to the operation inputting, thereby controlling the position and the posture of the endoscope 5001. Through the above-described control, it is possible to move the endoscope 5001 on the distal end of the arm unit 5031 from an optional position to another optional position, and then fixedly support the endoscope 5001 in the position after such movement. It is to be noted that the arm unit 5031 may be manipulated by a master-slave method. In this case, the arm unit 5031 may be remotely manipulated by the user through the input apparatus 5047 placed in a place away from an operating room.

Further, in a case where force control is applied, the arm controlling apparatus 5045 may perform so-called power assist control in which the arm controlling apparatus 5045 receives external force from the user and drives the actuators of the respective joint portions 5033a to 5033c to cause the arm unit 5031 to move smoothly in accordance with the external force. This enables the user to move the arm unit 5031 with relatively weak force when trying to move the arm unit 5031 while directly touching the arm unit 5031. Such a configuration allows the user to move the endoscope 5001 by a simpler manipulation more intuitively, offering improved convenience for the user.

The use of the supporting arm apparatus 5027 makes it possible to fix the position of the endoscope 5001 more reliably without depending on manpower. It is therefore possible to acquire an image of a surgical region in a stable manner and smoothly perform surgery.

It is to be noted that the arm controlling apparatus 5045 may not necessarily be disposed in the cart 5037. Furthermore, the number of apparatuses serving as the arm controlling apparatus 5045 may not necessarily be one. For example, the arm controlling apparatuses 5045 may be provided for each of the joint portions 5033a to 5033c of the arm unit 5031 of the supporting arm apparatus 5027, and a plurality of arm controlling apparatuses 5045 may cooperate with one another to control the driving of the arm unit 5031.

### (Light Source Apparatus 5043)

The light source apparatus 5043 supplies irradiation light for imaging the surgical region to the endoscope 5001. The light source apparatus 5043 includes, for example, a white light source including an LED, a laser light source, or a combination thereof. In this case, in a case where the white light source includes a combination of RGB laser light sources, it is possible to control output intensity and output timing precisely for each of colors (each of wavelengths), enabling white balance adjustment for a captured image in the light source apparatus 5043. In this case, it is also possible to perform time-divisional imaging for images respectively corresponding to RGB by performing time-divisional irradiation of the observation target with laser light from each of the RGB laser light sources and controlling driving of the imaging element in the camera head 5005 in synchronization with the irradiation timings. This method makes it possible to acquire a color image without providing color filters in the imaging element.

Furthermore, driving of the light source apparatus 5043 may be controlled to change the intensity of outputted light at predetermined time intervals. It is possible to generate a high dynamic range image free from so-called underexposed blocked up shadows and overexposed highlights by performing time-divisional image acquisition through control of the driving of the imaging element in the camera head 5005 in synchronization with the light intensity change timings and combining the thus acquired images.

Furthermore, the light source apparatus 5043 may be configured to supply light of a predetermined wavelength band ready for special light observation. In special light observation, for example, so-called narrow band observation (narrow band imaging: NBI) is performed, in which an image of predetermined tissue such as a blood vessel in a superficial portion of the mucous membrane is captured with high contrast through irradiation with light of a narrower band in comparison with irradiation light in ordinary observation (namely, white light) by utilizing the wavelength dependency of absorption of light in body tissue. Alternatively, in special light observation, fluorescent observation may be performed, in which an image is acquired using fluorescent light generated through irradiation with excitation light. In fluorescent observation, it is possible to perform observation of fluorescent light from body tissue by irradiating the body tissue with excitation light (autofluorescence observation) or to acquire a fluorescent light image by locally injecting a reagent such as indocyanine green (ICG) into body tissue and irradiating the body tissue with excitation light corresponding to a fluorescent light wavelength of the reagent. The light source apparatus 5043 may be configured to supply such narrow-band light and/or excitation light suitable for special light observation as described above.

### (Position Measurement Apparatus 6000)

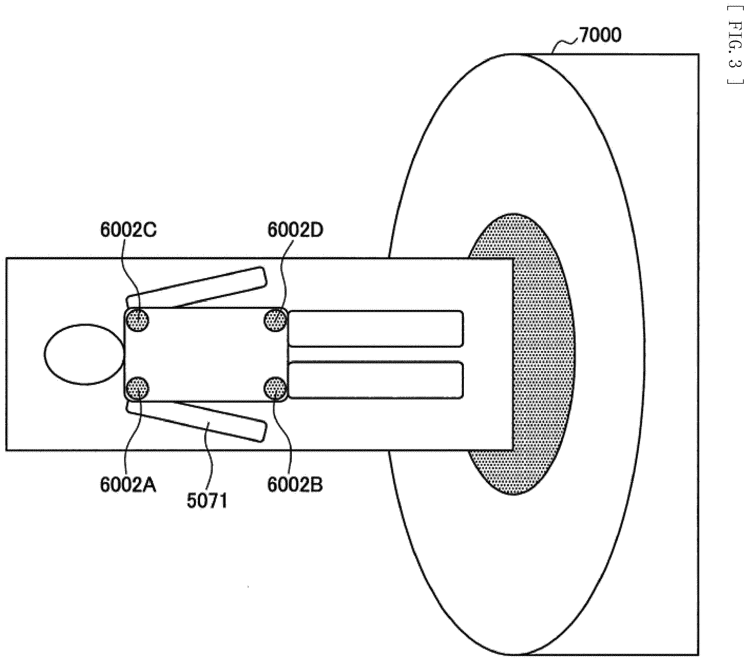

The position measurement apparatus 6000 measures the positions and the postures of the endoscope 5001 and the patient 5071 on the basis of a device marker 6001 attached to the endoscope 5001 and a patient marker 6002 attached to the patient 5071. The device marker 6001 may be attached to, for example, any of the surgical tools 5017 such as tweezers or a retractor as well as to the endoscope 5001. Furthermore, as illustrated in FIG. 3, the patient marker 6002 is attached to the patient 5071 also in a case where an element tissue information acquiring device 7000 that acquires three-dimensional distribution information of element tissue is used. The element tissue information acquiring device 7000 has a function of creating images of biological organs of the patient 5071, making it possible to acquire an element tissue image. In a case where the element tissue image is to be acquired before surgery, examples of the element tissue information acquiring device 7000 include CT, MRI, and the like. In a case where the element tissue image is to be acquired during surgery, examples of the element tissue information acquiring device 7000 include an ultrasonography device, an angiography device, an NBI device, and the like. As the patient marker 6002, as illustrated in FIG. 3, a marker 6002A, a marker 6002B, a marker 6002C, and a marker 6002D are attached to the body of the patient 5071. Thus, three-dimensional distribution information of element tissue located in the biological organs of the patient 5071 is acquired, and information related to a relative position of each of the biological organs and a relative position of the element tissue in the patient 5071 is acquired.

### <2-2. Configuration of Surgery System 1>

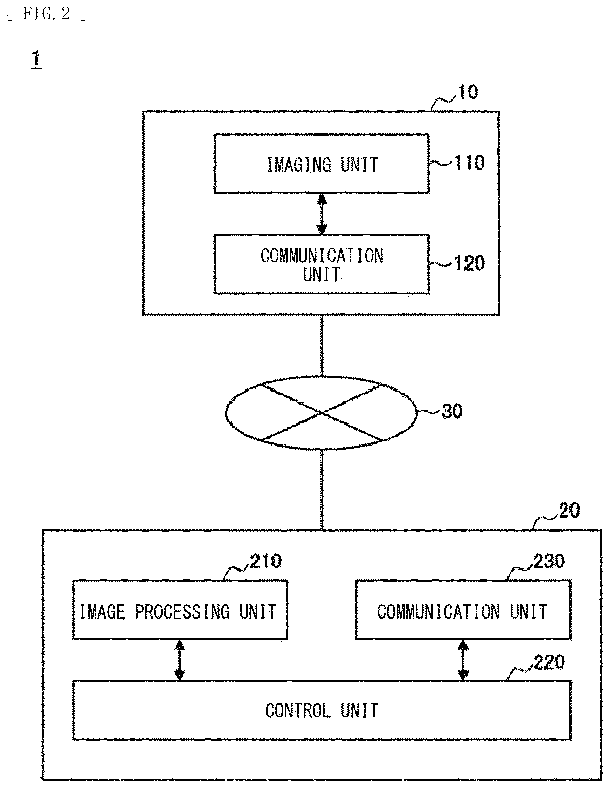

The following describes a configuration example of a surgery system 1 according to the embodiment of the present disclosure. FIG. 2 is a diagram illustrating the configuration example of the surgery system 1 according to the embodiment of the present disclosure. As illustrated in FIG. 2, the surgery system 1 according to the present embodiment includes a surgical camera 10 and an image processor 20 that are coupled to each other via a known network 30. It is to be noted that the network 30 may adopt a wired transmission cable such as an electrical signal cable ready for electrical signal communication, an optical fiber ready for optical communication, or a composite cable thereof. Alternatively, the network 30 may adopt a wireless communication method.

### (Surgical Camera 10)

The surgical camera 10 according to the present embodiment is an apparatus that performs imaging of a biological organ to acquire an image thereof. As the surgical camera 10 according to the present embodiment, for example, the above-described endoscope 5001 or an operation microscope may be used. Furthermore, the surgical camera 10 according to the present embodiment includes an imaging unit 110 and a communication unit 120.

### [Imaging Unit 110]

The imaging unit 110 has a function of performing imaging of a biological organ to acquire an image thereof. The imaging unit 110 according to the present embodiment therefore includes an imaging element such as a CCD (Charge Coupled Device) or a CMOS (Complementary MOS).

### [Communication Unit 120]

The communication unit 120 has a function of performing communication with the image processor 20 via the network 30. Specifically, the communication unit 120 transmits, to the image processor 20, an image of the interior of a biological body captured by the imaging unit 110. The communication unit 120 may also receive a control signal for controlling driving of the surgical camera 10 from the image processor 20.

### (Image Processor 20)

The image processor 20 according to the present embodiment specifies element tissue included in a three-dimensional region being a portion of the biological organ on the basis of three-dimensional distribution information of element tissue included in the biological organ, and superimposes and displays the specified element tissue on the image acquired by the surgical camera 10. The image processor 20 according to the present embodiment may include, for example, the CCU 5039 illustrated in FIG. 1. The image processor 20 according to the present embodiment includes an image processing unit 210, a control unit 220, and a communication unit 230.

### [Image Processing Unit 210]

The image processing unit 210 according to the present embodiment has a function of performing image processing on the image acquired from the surgical camera 10. As described above, the image processing unit 210 has a function of specifying element tissue included in a three-dimensional region being a portion of the biological organ on the basis of three-dimensional distribution information of element tissue included in the biological organ acquired by the element tissue information acquiring device 7000 as illustrated in FIG. 3, and superimposing and displaying the specified element tissue on the image acquired by the surgical camera 10.

### [Control Unit 220]

The control unit 220 performs control related to imaging of a surgical region by the surgical camera 10 and display of the image captured through the imaging. The control unit 220 also causes the display apparatus 5041 to display the image of the surgical region by a predetermined display method on the basis of an image signal that has gone through image processing by the image processing unit 210.

### [Communication Unit 230]

The communication unit 230 has a function of performing information communication with the surgical camera 10 via the network 30. Specifically, the communication unit 230 receives the image of the biological organ from the surgical camera 10.

Here, a method of superimposing the specified element tissue on the image acquired by the surgical camera 10 is described in detail.

First, the position measurement apparatus 6000 measures positions and postures of the surgical camera 10 and the patient 5071. Next, the endoscopic surgery system 5000 sets a surgical camera distal end coordinate system 6003 illustrated in FIG. 1 with respect to the measured position and the measured posture of the surgical camera 10. The endoscopic surgery system 5000 also sets a patient coordinate system 6004 with respect to the measured position and the measured posture of the patient 5071. The endoscopic surgery system 5000 then determines a transformation matrix for transforming the thus set patient coordinate system 6004 to the surgical camera distal end coordinate system 6003.

With use of the determined transformation matrix, the three-dimensional distribution information of the element tissue acquired by the element tissue information acquiring device 7000 is reflected in the image acquired by the surgical camera 10. Specifically, the patient marker 6002 is attached to the patient 5071 in a case where the element tissue information acquiring device 7000 acquires the three-dimensional distribution information of the element tissue. Specifically, as illustrated in FIG. 3, the marker 6002A, the marker 6002B, the marker 6002C, and the marker 6002D are attached to the body of the patient 5071. Then, the three-dimensional distribution information of element tissue located in biological organs of the patient 5071 corresponding to the patient coordinate system 6004 is acquired, and information related to a relative position of each of the biological organs and a relative position of the element tissue in the patient 5071 is acquired. The biological organs and the three-dimensional distribution information of the element tissue acquired on the patient coordinate system 6004 are transformed to the surgical camera distal end coordinate system 6003. Thus, on the image of the biological organ acquired by the surgical camera 10, a representation of element tissue included in a three-dimensional region being a portion of the biological organ is superimposed.

Subsequently, the image processing unit 210 performs image processing on an image signal transmitted from the surgical camera 10, and calculates the position and the shape of the biological organ, and changes in the position and the shape thereof. Specifically, an edge, a color, and the like of the biological organ are extracted from the image acquired from the surgical camera 10 as feature amounts, and the position and the shape of each biological organ and the position and the shape of the element tissue are calculated. Furthermore, the image processing unit 210 occasionally acquires images of the biological organ from the surgical camera 10 and detects changes in the feature amounts. Thus, the image processing unit 210 acquires information related to a change in the shape of the biological organ, the relative position of each biological organ, and/or the position and the shape of the element tissue. The image processing unit 210 may also determine a direction of imaging of the biological organ from the color, the shape, and the like of the biological organ. As described above, the image processing unit 210 is able to detect, for example, a positional relationship between the element tissue and the biological organ, surrounding tissue, and an ischemic boundary resulting from portal vein ligation, and a change therein. As a result, the position of the element tissue to be superimposed on the image acquired by the surgical camera 10 is adjusted according the change in the shape of the biological organ by excision thereof or the like. With the element tissue superimposed on the image acquired by the surgical camera 10 as described above, for example, it is possible to identify subsegments of the liver more exactly using hepatic veins and the ischemic boundary as characteristics. Furthermore, it is possible to perform systematic hepatectomy involving excision of the liver in each subsegment more smoothly.

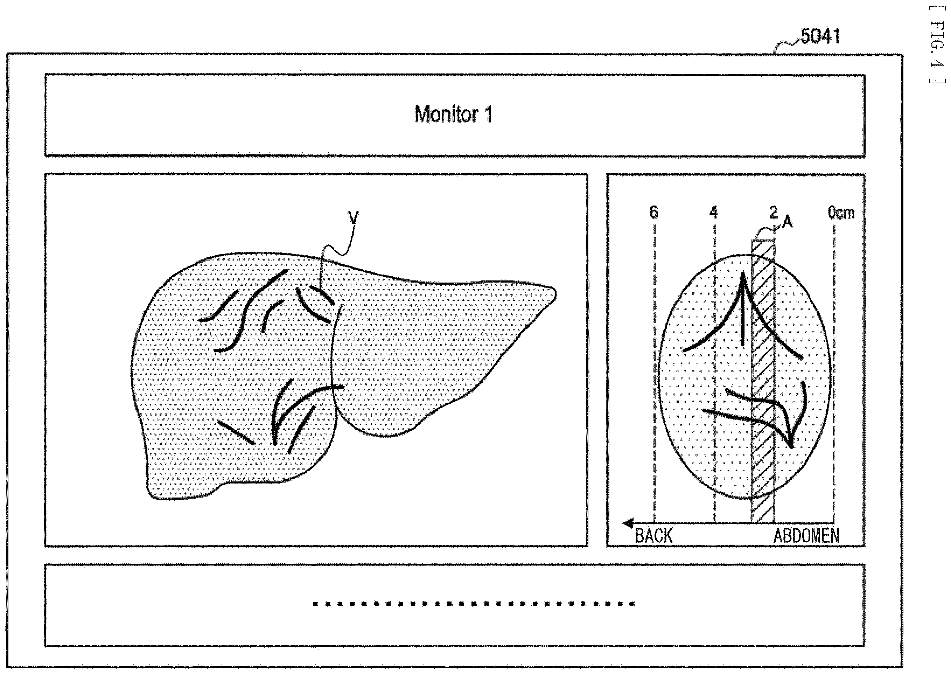

Furthermore, the control unit 220 causes the display apparatus 5041 to display the thus acquired superimposition image by a predetermined display method. The following describes a method for displaying the superimposition image with reference to FIGs. 4 to 6.

FIG. 4 is a schematic diagram illustrating an example of the superimposition image displayed on the display apparatus 5041. In FIG. 4, a representation of blood vessels, which are a type of element tissue V, is superimposed and displayed on an image of the liver, which is a type of biological organs. The control unit 220 provides the display apparatus 5041 with a first image illustrated in a left portion of FIG. 4, which is an image of the biological organ viewed from a first direction, and a second image illustrated in a right portion of FIG. 4, which is an image of the biological organ viewed from a second direction orthogonal to the first direction. The second image may be a reference image exhibiting locations along the first direction. In FIG. 4, the first direction is a direction from the abdomen toward the back of the patient 5071. A coordinate axis along the first direction, for example, may be displayed on the second image illustrated in the right portion of FIG. 4. Furthermore, a region A including the element tissue to be superimposed may be displayed on the second image. The representation of the element tissue included in the region A is superimposed and displayed on the first image. With the element tissue superimposed and displayed on the first image, it is possible for a user to perform surgery without switching the image acquired by the surgical camera 10 to another image.

Furthermore, a position and a range of the region A may be displayed on the second image. For example, a scale along the first direction may be displayed as illustrated in FIG. 4. The display of the position and the range of the region A is not limited to display of a scale, and it is sufficient if the user perceives the position and the range of the region A. For example, coordinate values or the like of the region A may be displayed.

Furthermore, the position and the range of the region A may be set as appropriate by the user. The user selectively displays desired element tissue by setting the position and the range of the region A. As a result, it is possible to reduce the risk of, for example, significant bleeding due to a mistakenly cut blood vessel, dissemination due to a wound of a malignant tumor, and dysfunction due to nervous plexus injury in excision of the biological organ.

A method of setting the position and the range of the region A by the user may be performed by using a known user interface. For example, a user interface such as the foot switch 5057 illustrated in FIG. 1, a touch panel, a speech recognition technology, a gesture recognition technology, a proximity sensor, a joystick attached to any of the surgical tools 5017, and a physical button may be used. It is to be noted that for example various other surgery assisting information such as heart rate or amount of bleeding may be displayed on the display apparatus 5041 in addition to the first image and the second image.

In addition, the position of the region A may be determined in accordance with a position of a predetermined surgical tool 5017. For example, the position measurement apparatus 6000 may detect a position of a distal end of the energy device 5021 being handled by the user to use the detected position of the distal end of the energy device 5021 as a reference position, and a range preset with respect to the reference position may be determined as the region A. In this case, the position of the energy device 5021 may be detected on the basis of the device marker 6001 attached to the energy device 5021. This enables the user to know element tissue such as blood vessels to be particularly careful about during the surgery.

The control unit 220 may display a portion of the superimposed and displayed element tissue in an emphasized manner in accordance with a predetermined surgical tool 5017. For example, the element tissue may be superimposed and displayed by the method described above, the position of the distal end of the energy device 5021 may be regarded as a reference position by the above-described method, and the control unit 220 may display element tissue located within a range predetermined with respect to the reference position in an emphasized manner.

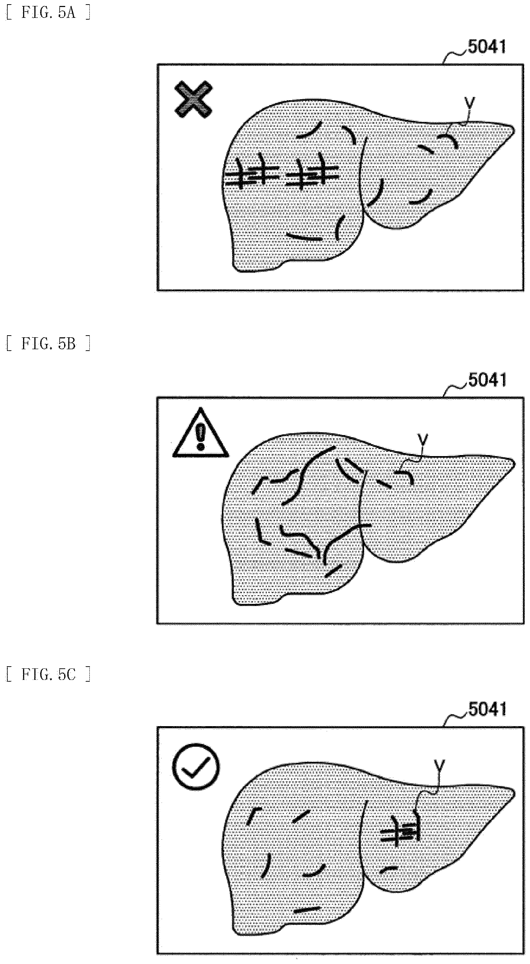

The control unit 220 may further perform control to display the element tissue and the location of the element tissue along the first direction in a relative manner. The control unit 220 may set, for example, the direction from the abdomen toward the back of the patient 5071 as the first direction, divide the biological organ into three sections along the first direction, and superimpose and display element tissue included in each of the sections of the biological organ. For example, FIG. 5A illustrates an image acquired by superimposing and displaying the element tissue V included in the biological organ divided into three sections. FIG. 5B illustrates an image acquired by superimposing and displaying the element tissue V included in a second section of the biological organ from the abdomen side out of the three sections of the biological organ. FIG. 5C illustrates an image acquired by superimposing and displaying the element tissue V included in the section of the biological organ on the back side out of the three sections of the biological organ. The control unit 220 may display, on each of the images, an icon corresponding to the location of each of the sections in the biological organ. For example, an icon indicating that the element tissue V is in a location at a shallow depth is displayed in an upper left portion of FIG. 5A, an icon indicating that the element tissue V is in a location at a relatively shallow depth is displayed in an upper left portion of FIG. 5B, and an icon indicating that the element tissue V is in a location at a deep depth is displayed in an upper left portion of FIG. 5C. This allows the surgeon 5067 to proceed with surgery while checking the displayed icons as appropriate.

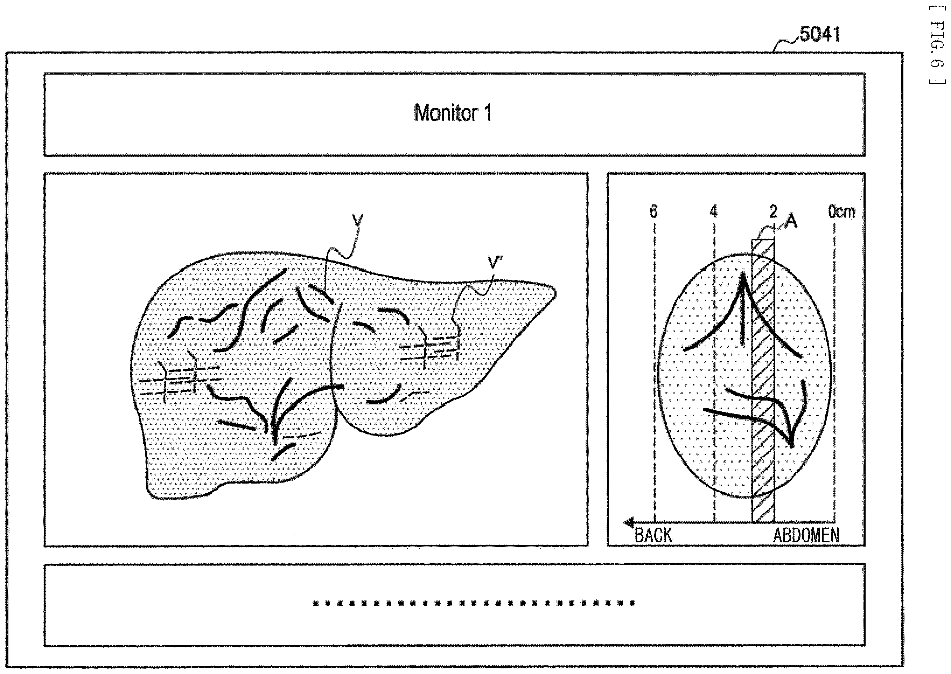

The control unit 220 may further perform control to display, on the second image, element tissue V' located in a portion of the biological organ that is not included in the region A. The control unit 220 may perform, for example, control to display the element tissue V included in the region A and the element tissue V' located in the portion of the biological organ that is not included in the region A in different manners as illustrated in a left portion of FIG. 6. For example, the element tissue V may be displayed in an emphasized manner, whereas the element tissue V' located away from the region A may be displayed in a manner that color and brightness or blurriness of the image thereof change along the first direction using brightness of the element tissue V as a reference. As a result, the user readily knows the distance of the element tissue V' from the region A.

Furthermore, the control unit 220 may simultaneously superimpose and display a plurality of types of element tissue selected by the user on the image acquired by the surgical camera 10. For example, among a blood vessel, a nervous plexus, a lymphatic vessel, and a tumor, the control unit 220 may simultaneously superimpose and display a blood vessel and a tumor as two types of element tissue on the image acquired by the surgical camera 10. As described above, the control unit 220 may superimpose and display at least one type of element tissue, among the plurality of types of element tissue, on the image acquired by the surgical camera 10. Furthermore, the control unit 220 may superimpose and display predetermined element tissue in a type of element tissue selected by the user, on the image acquired by the surgical camera 10. For example, in a case where the element tissue to be superimposed and displayed is blood vessels, the image processing unit 210 detects a difference in feature amounts between an artery and a vein, and the control unit 220 may superimpose and display, for example, only a representation of the artery on the image acquired from the surgical camera 10. For another example, the control unit 220 may cause the display apparatus 5041 to selectively superimpose and display blood vessels for each of segments of the biological organ (for example, for each of subsegments of the liver) or to selectively superimpose and display only an anatomically significant blood vessel. Superimposing only element tissue desired by the user and displaying such element tissue on the display apparatus 5041 as described above makes it possible for the surgery system 1 to reduce a burden on the user.

### <3. Operation>

The following describes an example of operations of the surgery system 1 in detail.

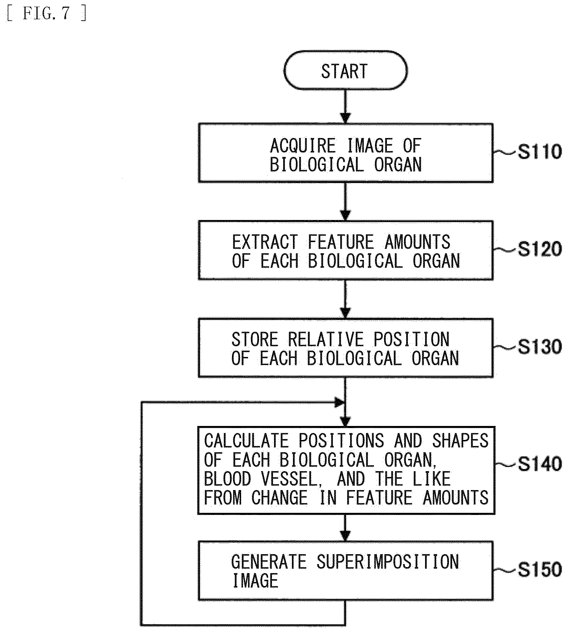

The operations of the surgery system 1 according to the present embodiment are described with reference to FIGs. 7 to 9. FIG. 7 is a flowchart illustrating an example of a flow of an operation of the surgery system 1 according to the present embodiment.

First, the surgical camera 10 acquires an image of a biological organ of the patient 5071 (S110). The image acquired by the surgical camera 10 is transmitted to the image processor 20 via the network 30. Next, the image processing unit 210 included in the image processor 20 performs image processing on the image transmitted from the surgical camera 10 and extracts an edge, a color, and the like of the biological organ as feature amounts thereof (S120). The image processing unit 210 calculates the shape of the biological organ from the feature amounts thereof, and further calculates and stores the relative position of each biological organ (S130). Next, the image processing unit 210 performs image processing on the image acquired from the surgical camera 10, extracts feature amounts of element tissue, and calculates the position and the shape of the element tissue (S140). The control unit 220 included in the image processor 20 then generates a superimposition image by superimposing a representation of the element tissue on the image acquired by the surgical camera 10 (S150). Thereafter, processes in S140 and S150 are repeated. The image processing unit 210 occasionally acquires images of the biological organ from the surgical camera 10 and performs image processing on the acquired images as appropriate to extract a change in the feature amounts. The image processing unit 210 then calculates changes in the positions and the shapes of the biological organ and the element tissue that occur during surgery, and updates the superimposition image being displayed on the display apparatus 5041.

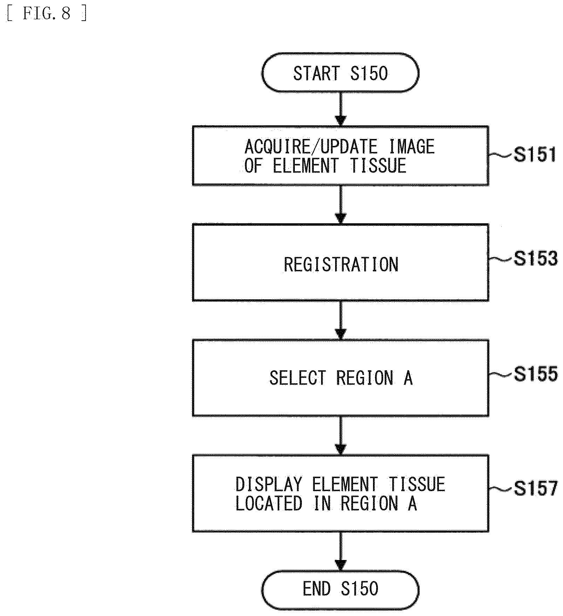

Here, the process in S150 is described in detail with reference to FIG. 8. First, the element tissue information acquiring device 7000 acquires an image of element tissue in a biological organ (S151). The acquired image of the element tissue is saved as three-dimensional distribution information of the element tissue in the biological organ. The three-dimensional distribution information of the image of the element tissue in the biological organs may be acquired, for example, using the element tissue information acquiring device 7000 before or during the surgery.

Next, the control unit 220 reflects the acquired three-dimensional distribution information of the element tissue in the image of the biological organ acquired by the surgical camera 10 and registers the information with the image (S153). Specifically, the position measurement apparatus 6000 measures the positions and/or the postures of the surgical camera 10 and the patient 5071. Next, the endoscopic surgery system 5000 sets the surgical camera distal end coordinate system 6003 illustrated in FIG. 1 with respect to the measured position and the measured posture of the surgical camera 10. The endoscopic surgery system 5000 also sets the patient coordinate system 6004 with respect to the measured position and the measured posture of the patient 5071. The endoscopic surgery system 5000 determines a transformation matrix for transforming the thus set patient coordinate system 6004 to the surgical camera distal end coordinate system 6003. With use of the determined transformation matrix, the control unit 220 transforms the three-dimensional distribution information of the biological organ and the element tissue acquired on the patient coordinate system 6004 to the surgical camera distal end coordinate system 6003.

Next, a user selects the position and the range of the region A in which a representation of element tissue is superimposed on the image acquired by the surgical camera 10 (S155). At this time, the user may set the first direction for selecting the region A. For example, the first direction may be the direction from the abdomen toward the back of the patient 5071.

Then, element tissue included in the region A is superimposed and displayed on the image of the biological organ acquired by the surgical camera 10 (S157). The above-described processes are performed as appropriate every time the image acquired by the surgical camera 10 is updated.

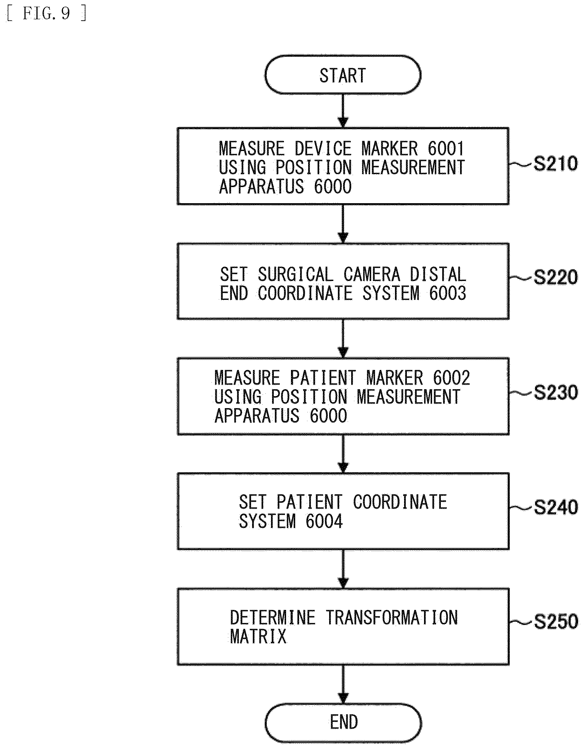

The following describes a measurement method of the positions and the postures of the surgical camera 10 and the patient 5071, and a coordinate transformation method with the use of the position measurement apparatus 6000 with reference to FIG. 9. First, the device marker 6001 for the measurement of the position and the posture is attached to the surgical camera 10, and the position measurement apparatus 6000 measures the position of the device marker 6001 (S210). No particular limitations are placed on the location to which the device marker 6001 is attached as long as it is possible to specify the position of the surgical camera 10. For example, the device marker 6001 may be attached to the camera head 5005 or the arm unit 5031. Next, the endoscopic surgery system 5000 sets the surgical camera distal end coordinate system 6003 on the basis of the device marker 6001 (S220).

Next, the patient marker 6002 for the measurement of the position and the posture of the patient 5071 is attached to the patient 5071, and the position measurement apparatus 6000 measures the position of the patient marker 6002 (S230). No particular limitations are placed on the location to which the patient marker 6002 is attached as long as it is possible to specify the position and the posture of the patient 5071. Preferably, the patient marker 6002 is attached to a location where the position of the surgical region and a change in the shape thereof are acquired more exactly. Next, the endoscopic surgery system 5000 sets the patient coordinate system 6004 on the basis of the patient marker 6002 (S240). The endoscopic surgery system 5000 then determines a transformation matrix for transforming the thus set patient coordinate system 6004 to the surgical camera distal end coordinate system 6003 (S250). The patient coordinate system 6004 is transformed to the surgical camera distal end coordinate system 6003 using the transformation matrix.

### <4. Hardware Configuration>

The embodiment of the present disclosure has been described above. The above-described image processing is implemented through cooperation between software and hardware of an information processor described below.

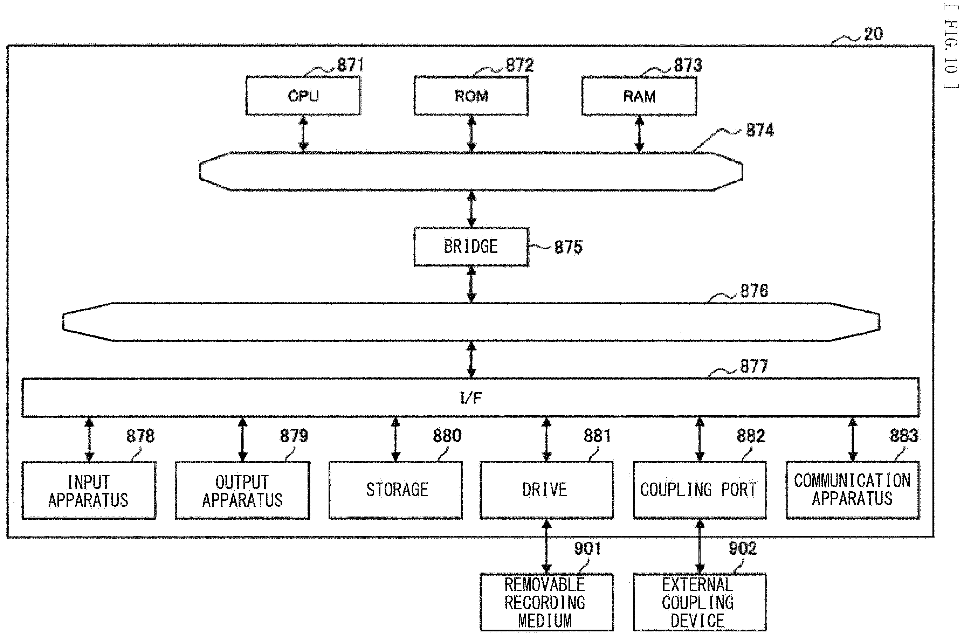

The following describes an example of a hardware configuration of the image processor 20 according to an embodiment of the present disclosure. FIG. 10 is a block diagram illustrating the example of the hardware configuration of the image processor 20 according to the embodiment of the present disclosure. Referring to FIG. 10, the image processor 20 includes, for example, a CPU 871, a ROM 872, a RAM 873, a host bus 874, a bridge 875, an external bus 876, an interface 877, an input apparatus 878, an output apparatus 879, a storage 880, a drive 881, a coupling port 882, and a communication apparatus 883. It is to be noted that the hardware configuration described herein is an example, and some of constituent elements therein may be omitted. Furthermore, a constituent element other than those that are described herein may be further included.

### (CPU 871)

The CPU 871 functions as, for example, an arithmetic processor or a controller, and controls the entirety or a portion of an operation of each of the constituent elements on the basis of various programs recorded on the ROM 872, the RAM 873, the storage 880, or a removable recording medium 901.

### (ROM 872 and RAM 873)

The ROM 872 is a means for storing therein a program to be read by the CPU 871, data to be used for arithmetic operation, and the like. The RAM 873 temporarily or permanently stores therein, for example, a program to be read by the CPU 871, various parameters that are changed as appropriate in execution of the program, and the like.

### (Host Bus 874, Bridge 875, External Bus 876, and Interface 877)

The CPU 871, the ROM 872, and the RAM 873 are coupled to one another via the host bus 874 enabled for high-speed data transmission, for example. Meanwhile, the host bus 874 is coupled to the external bus 876 having a relatively low data transmission speed via the bridge 875, for example. Furthermore, the external bus 876 is coupled to various constituent elements via the interface 877.

### (Input Apparatus 878)

As the input apparatus 878, for example, a mouse, a keyboard, a touch panel, buttons, a switch, a lever, and the like are used. Furthermore, a remote controller (referred to below as a remote) enabled to transmit a control signal using infrared or other radio waves may be used as the input apparatus 878. Furthermore, the input apparatus 878 includes a voice input apparatus such as a microphone.

### (Output Apparatus 879)

The output apparatus 879 is an apparatus enabled to visually or aurally notify the user of acquired information. Examples thereof include a display apparatus such as a CRT (Cathode Ray Tube) display, a LCD, or an organic EL display, an audio output apparatus such as a speaker or a headphone, a printer, a mobile phone, a facsimile machine, or the like.

### (Storage 880)

The storage 880 is an apparatus for storing various types of data. As the storage 880, for example, a magnetic storage device such as a hard disk drive (HDD), a semiconductor storage device, an optical storage device, and a magneto-optical storage device.

### (Drive 881)

The drive 881 is, for example, an apparatus that reads out information recorded on the removable recording medium 901 such as a magnetic disk, an optical disk, a magneto-optical disk, or semiconductor memory, or writes information to the removable recording medium 901.

### (Removable Recording Medium 901)

Examples of the removable recording medium 901 include a DVD medium, a Blu-ray (registered trademark) medium, an HD DVD medium, and various types of semiconductor storage media. Needless to say, the removable recording medium 901 may be, for example, an IC card containing a non-contact IC chip, an electronic device, or the like.

### (Coupling Port 882)

The coupling port 882 is, for example, a port for coupling an external coupling device 902. Examples thereof include a USB (Universal Serial Bus) port, an IEEE 1394 port, a SCSI (Small Computer System Interface), an RS-232C port, an optical audio terminal, or the like.

### (External Coupling Device 902)

The external coupling device 902 is, for example, a printer, a portable music player, a digital camera, a digital video camera, an IC recorder, or the like.

### (Communication Apparatus 883)

The communication apparatus 883 is a communication device for coupling to a network, and examples thereof include a communication card for a wired or wireless LAN, Bluetooth (registered trademark), or WUSB (Wireless USB), a router for optical communication, an ADSL (Asymmetric Digital Subscriber Line) router, a modem for various types of communication, or the like.

### <5. Conclusion>

A preferred embodiment(s) of the present disclosure has/have been described above in detail with reference to the accompanying drawings, but the technical scope of the present disclosure is not limited to such an example(s). It is apparent that a person having ordinary skill in the art of the present disclosure can arrive at various alterations and modifications within the scope of the technical idea described in the appended claims, and it is understood that such alterations and modifications naturally fall within the technical scope of the present disclosure.

Furthermore, the effects described herein are merely illustrative and exemplary, and not limiting. That is, the technology according to the present disclosure can exert other effects that are apparent to those skilled in the art from the description herein, in addition to the above-described effects or in place of the above-described effects.

It is to be noted that the following configurations are also fall within the technical scope of the present disclosure.
(1) A surgery system including:
   a surgical camera that performs imaging of a biological organ to acquire an image of the biological organ; and
   an image processor that specifies element tissue included in a three-dimensional region being a portion of the biological organ during surgery on the basis of three-dimensional distribution information of element tissue included in the biological organ and superimposes a representation of the specified element tissue on the image of the biological organ.
(2) The surgery system according to (1), in which the three-dimensional region includes a region of the biological organ located on a range of a portion in a first direction.
(3) The surgery system according to (2), in which the image processor generates a range representation indicating a range of the three-dimensional region in the first direction.
(4) The surgery system according to (3), in which
   the image processor further generates a reference image exhibiting the biological organ viewed from a second direction orthogonal to the first direction, and
   the range representation includes a representation indicating the range on the reference image.
(5) The surgery system according to (3), in which
   the image processor further generates a reference image exhibiting the biological organ viewed from a second direction orthogonal to the first direction,
   a scale along the first direction is displayed on the reference image, and
   the range representation includes a representation of numerical values indicating the range in the first direction.
(6) The surgery system according to any one of (1) to (5), in which the three-dimensional region includes a region of a portion of the biological organ selected by a user.
(7) The surgery system according to any one of (1) to (5), in which the three-dimensional region includes a region of a portion of the biological organ determined in accordance with a position of a predetermined surgical tool.
(8) The surgery system according to any one of (1) to (7), in which the image processor displays a portion of the representation of the element tissue in an emphasized manner, the portion corresponding to a position of a predetermined surgical tool.
(9) The surgery system according to any one of (1) to (8), in which the image processor superimposes, on the image of the biological organ, a representation of at least one of a blood vessel, a nervous plexus, a lymphatic vessel, or a tumor as the element tissue.
(10) The surgery system according to any one of (1) to (9), in which
   the biological organ includes a liver, and
   the image processor superimposes a representation of a blood vessel as the element tissue on an image of the liver.
(11) The surgery system according to any one of (1) to (10), in which
   the image processor further superimposes, on the image of the biological organ, a representation of element tissue included outside the three-dimensional region, and
   the representation of the element tissue included outside the three-dimensional region and the representation of the element tissue included in the three-dimensional region are displayed in different manners.
(12) The surgery system according to any one of (1) to (11), in which
   the biological organ includes a plurality of types of element tissue, and
   the image processor superimposes, on the image of the biological organ, a representation of a type of element tissue selected by a user from among the plurality of types of element tissue.
(13) An image processor including:
   an image processing unit that specifies element tissue included in a three-dimensional region being a portion of a biological organ during surgery on the basis of three-dimensional distribution information of element tissue included in the biological organ and superimposes a representation of the specified element tissue on an image of the biological organ acquired by a surgical camera.
(14) An image processing method including:
   implementation by a processor of specifying, element tissue included in a three-dimensional region being a portion of a biological organ during surgery on the basis of three-dimensional distribution information of element tissue included in the biological organ, and superimposing a representation of the specified element tissue on an image of the biological organ acquired by a surgical camera.

### Reference Signs List

- 1:: Surgery system
- 10:: Surgical camera
- 20:: Image processor
- 30:: Network
- 110:: Imaging unit
- 120, 230:: Communication unit
- 210:: Image processing unit
- 5001:: Endoscope
- 5039:: CCU
- 5041:: Display apparatus
- 6000:: Position measurement apparatus
- 6001:: Device marker
- 6002:: Patient marker
- 6003:: Surgical camera distal end coordinate system
- 6004:: Patient coordinate system

## Claims

1. A surgery system comprising:
a surgical camera that performs imaging of a biological organ to acquire an image of the biological organ; and
an image processor that specifies element tissue included in a three-dimensional region being a portion of the biological organ during surgery on a basis of three-dimensional distribution information of element tissue included in the biological organ and superimposes a representation of the specified element tissue on the image of the biological organ.

2. The surgery system according to claim 1, wherein the three-dimensional region includes a region of the biological organ located on a range of a portion in a first direction.

3. The surgery system according to claim 2, wherein the image processor generates a range representation indicating a range of the three-dimensional region in the first direction.

4. The surgery system according to claim 3, wherein the image processor further generates a reference image exhibiting the biological organ viewed from a second direction orthogonal to the first direction, and
the range representation includes a representation indicating the range on the reference image.

5. The surgery system according to claim 3, wherein
the image processor further generates a reference image exhibiting the biological organ viewed from a second direction orthogonal to the first direction,
a scale along the first direction is displayed on the reference image, and
the range representation includes a representation of numerical values indicating the range in the first direction.

6. The surgery system according to claim 1, wherein the three-dimensional region includes a region of a portion of the biological organ selected by a user.

7. The surgery system according to claim 1, wherein the three-dimensional region includes a region of a portion of the biological organ determined in accordance with a position of a predetermined surgical tool.

8. The surgery system according to claim 1, wherein the image processor displays a portion of the representation of the element tissue in an emphasized manner, the portion corresponding to a position of a predetermined surgical tool.

9. The surgery system according to claim 1, wherein the image processor superimposes, on the image of the biological organ, a representation of at least one of a blood vessel, a nervous plexus, a lymphatic vessel, or a tumor as the element tissue.

10. The surgery system according to claim 1, wherein
the biological organ includes a liver, and
the image processor superimposes a representation of a blood vessel as the element tissue on an image of the liver.

11. The surgery system according to claim 1, wherein
the image processor further superimposes, on the image of the biological organ, a representation of element tissue included outside the three-dimensional region, and
the representation of the element tissue included outside the three-dimensional region and the representation of the element tissue included in the three-dimensional region are displayed in different manners.

12. The surgery system according to claim 1, wherein
the biological organ includes a plurality of types of element tissue, and
the image processor superimposes, on the image of the biological organ, a representation of a type of element tissue selected by a user from among the plurality of types of element tissue.

13. An image processor comprising:
an image processing unit that specifies element tissue included in a three-dimensional region being a portion of a biological organ during surgery on a basis of three-dimensional distribution information of element tissue included in the biological organ and superimposes a representation of the specified element tissue on an image of the biological organ acquired by a surgical camera.

14. An image processing method comprising:
implementation by a processor of specifying, element tissue included in a three-dimensional region being a portion of a biological organ during surgery on a basis of three-dimensional distribution information of element tissue included in the biological organ, and superimposing a representation of the specified element tissue on an image of the biological organ acquired by a surgical camera.
